# EUROPEAN PATENT SPECIFICATION

(11) **EP 2 043 625 B1**
(45) Date of publication and mention of the grant of the patent: **01.08.2012**
(21) Application number: 07798778.2
(22) Date of filing: 19.06.2007
(51) Int. Cl.: A61K 31/192, A61K 31/352, A61K 31/366, A61K 31/381, A61K 31/401, A61K 31/4168, A61K 31/4402, A61K 31/498, A61P 25/02

(54) **NFKAPPAB INHIBITORS TO TREAT PAIN LOCALLY**
NFKAPPAB-HEMMER ZUR LOKALEN SCHMERZBEHANDLUNG
INHIBITEURS NFKAPPAB POUR LE TRAITEMENT LOCAL DE LA DOULEUR

(30) Priority: 26.07.2006 US 460012
(43) Date of publication of application: 08.04.2009
(60) Divisional application: 11168573.1
(73) Proprietor: Medtronic, Inc., Minneapolis, MN 55432-5604 (US)
(72) Inventor: BURRIGHT, Eric, N., Eagan, MN 55123 (US); SHAFER, Lisa, L., Stillwater, MN 55082 (US); MCKAY, Bill, Memphis, TN 38133 (US); ZANELLA, John, Cordova, TN 38018 (US)
(74) Representative: O'Connell, Maura
(86) International application number: PCT/US2007/071593
(87) International publication number: WO 2008/014066

(56) References cited:
- EP-A- 1 405 646
- WO-A-2004/039355
- US-A- 5 801 188
- US-A1- 2004 265 364
- US-A1- 2005 095 246

## Description

### FIELD OF THE INVENTION

The present invention relates to systems and methods for contributing to the local treatment of pain. More specifically, the systems and methods of the present Invention contribute to the local treatment of pain by inhibiting the NFκB family of transcription factors.

### BACKGROUND OF THE INVENTION

Pain can be divided into two types: acute pain and neuropathic pain. Acute pain refers to pain experienced when tissue is being damaged or is damaged. Acute pain serves at least two physilologically advantageous purposes. First, it warns of dangerous environmental stimuli (such as hot or sharp objects) by triggering reflexive responses that end contact with the dangerous stimuli. Second, if reflexive responses do not avoid dangerous environmental stimuli effectively, or tissue injury or infection otherwise results, acute pain facilitates recuperative behaviors. For example, acute pain associated with an injury or infection encourages an organism to protect the compromised area from further Insult or use while the injury or infection heals. Once the dangerous environmental stimulus is removed, or the injury or infection has resolved, acute pain, having served its physiological purpose, ends.

As contrasted to acute pain, neuropathic pain serves no beneficial purpose. Neuropathic pain results when pain associated with an injury or infection continues in an area once the injury or infection has resolved. The biological basis for this type of pain that exists in the absence of physical injury or infection baffled scientists for many years. Recently, however, evidence has mounted that neuropathic pain is caused, at least in part, by on-going (and unneeded) activation of the immune system after an injury or infection has healed. See, for example, WATKINS & MAINER (2004), PAIN, CLINICAL UPDATES, 1-4.

Local immune system activation begins when damaged cells secrete signals that recruit immune system cells to the area. One type of recruited immune system cell is the macrophage. Macrophages release interleukin-1 beta ("IL-1β"), interleukin-6 ("IL-6") and tumor necrosis factor alpha ("TNFα"), pro-inflammatory cytokines heavily involved in orchestrating the immediate and local physiological effects of injury or infection. For instance, once released, pro-inflammatory cytokines promote inflammation (swelling and redness caused by increased blood flow to the area which delivers recruited immune system cells more quickly) and also increased sensitivity to pain (by increasing the excitability and transmission of sensory nerves carrying pain information to the brain). Thus, pro-inflammatory cytokines are involved in the beneficial physiological and recuperative effects of acute pain.

Normally after an injury or infection heals, the local immune system response ceases, inflammation recedes and the increased sensitivity to pain abates. In some individuals, however, signals that terminate the immune system response are not entirely effective and pro-inflammatory cytokine activity in the area remains active. In these individuals, sensory nerves carrying pain information to the brain remain sensitized in the absence of injury or infection and the individuals can experience neuropathic pain.

Sciatica provides an example of pain that can transition from acute to neuropathic pain. Sciatica refers to pain associated with the sciatic nerve which runs from the lower part of the spinal cord (the lumbar region), down the back of the leg and to the foot. Sciatica may be caused by a herniated disc. The herniated disc itself leads to local immune system activation. The herniated disc also may damage the nerve root by pinching or compressing it, leading to additional immune system activation in the area. In most individuals, the acute pain and immune system activation associated with the injury cease once the damage has been repaired. In those individuals where immune system activation does not abate completely, however, neuropathic pain may result.

As the foregoing suggests, inhibiting the actions of pro-inflammatory cytokines can provide an effective strategy for treating acute and neuropathic pain. Inhibiting the immune system, however, is problematic as a general treatment because it leaves an individual vulnerable to infection and unable to repair tissue injuries effectively. Thus, systemic treatments that inhibit pro-inflammatory cytokines throughout the body generally are not appropriate except in the most extreme cases of neuropathic pain. Other pain treatments likewise are not effective or appropriate for treating acute or neuropathic pain caused by pro-inflammatory cytokines. For example, narcotics do not treat pain mediated by the pro-inflammatory cytokines because narcotics block opiate receptors, a receptor type not directly involved in many effects of the pro-inflammatory cytokines. A need exists, therefore, for a locally-administered pain treatment that suppresses the actions of the pro-inflammatory cytokines.

Generally, for a protein such as a pro-inflammatory cytokine to exert an effect, the cell that will use or secrete the protein must create it. To create a protein the cell first makes a copy of the protein's gene sequence in the nucleus of the cell (this process is called transcription). Transcription factors are regulatory proteins that initiate the transcription process upon binding with DNA. Following transcription, the newly made copy of the gene sequence that encodes for the protein (called messenger RNA ("mRNA")) leaves the nucleus and is trafficked to a region of the cell containing ribosomes. Ribosomes assist in reading the sequence of the mRNA and create the protein for which it encodes. This process of new protein synthesis is known as translation. A variety of factors affect the rate and efficiency of transcription and translation. One of these factors includes the intracellular regulation of transcription factors.

The NFκB family is one group of transcription factors that plays an essential role in the inflammatory response through transcriptional regulation of a variety of genes encoding pro-inflammatory cytokines (TNFα, IL-1β, IL-6), chemokines (IL-8, MIP1α), inducible effector enzymes (iNOS and COX-2), and other molecules. Pro-inflammatory cytokines that are up-regulated by NFκB, such as TNFα and IL-1β, can also directly activate the NFκB pathway, thus establishing an autoregulatory loop that can result in chronic Inflammation and pain. Activation of NFκB pathways has been shown to be important in the pathogenesis of many chronic inflammatory diseases including rheumatoid arthritis, inflammatory bowel disease, osteoarthritis, osteolysis, tendonitis, sciatica, herniated disc, stenosis, mylopathy, low back pain, facet pain, tendonitis, carpal tunnel syndrome, tarsal tunnel syndrome, and failed back syndrome.

Thus, NFκB pathway inhibition is an attractive therapeutic strategy for the treatment of inflammatory and pain disorders. Effective NFκB pathway blockade could result in lower levels of an array of molecules including pro-inflammatory cytokines that contribute to inflammation and pain. However, because NFκB is also involved in normal cellular physiology, such as mounting an effective immune response, systemic inhibition of this pathway could result in serious side effects. For these reasons, minimizing systemic exposure of animals to NFκB inhibitory compounds is an important component of a safe therapeutic strategy.

### PRIOR ART

US 2005/095246 (Shafer) describes methods and devices to attenuate tumor necrosis factor (TNF) and other pro-inflammatory mediators in the CNS to treat neurological, neurodegenerative, neuropsychiatric disorders, pain and brain injury. More particularly, TNF blocking agents that target intracellular signals and downstream effects associated with the production and secretion of TNF are described. Devices described include therapy delivery devices comprising a reservoir capable of housing a TNF blocking agent and a catheter operably coupled to the device and adapted to deliver the TNF blocking agent to a target site within a subject. Shafer refers specifically to various TNF modifying agents, including a pyrrolidine dithiocarbamatem (PDTC) derivative and the IKK-B inhibitor referenced as BMS 345541. A compound may be administered perispinally.

US 5,801,188 (Hassenbusch III) discloses methods for intraspinal administration of therapeutically-effective amounts of clonidine for alleviating acute and chronic pain.

WO 2004/039355 (Polymerix Corporation) describes formulations of injectable particles (e.g., microspheres) which can be used for intra-articular injection. These formulations are made of biocompatible polymers that biodegrade to generate NSAIDs, and are useful for treating inflamed joints, thus providing safe, long-lasting relief of joint pain and swelling.

US 2004/065364 (Oz turk) describes compositions for transdermal pain relief comprising amitriptyiline, clonidine, ketamine and an anti-inflammatory in a base.

EP 1 405 646 A2 (Fang-Yu) describes a therapy for the treatment of pain comprising a diclofenac salt of lidocaine for the treatment of pain, particularly by topical or parenteral administration. The diclofenac portion of the salt may be replaced by another NSAID and the lidocaine portion may be replaced with another local anesthetic.

### SUMMARY OF THE INVENTION

Embodiments according to the present invention can treat pain through the local administration of one or more compounds that inhibit the NFκB pathway. Local administration of these compounds helps to prevent unwanted side effects, such as immunosuppression, associated with systemic drug administration.

Specifically, one embodiment according to the present invention is an NRκB inhibiting compound for use in the treatment of pain associated with sciatica or herniated disc or radicular pain by administration locally to a patient in need thereof wherein the inhibitor is selected from sulfasalazine or sulindac or combinations thereof. The administering of the NFκB inhibiting compounds can inhibit the production of one or more pro-inflammatory cytokines selected from the group consisting of interleukin 1 beta (IL-1β), tumor necrosis factor alpha (TNFα) and interleukin-6 (IL-6).

In accordance with the present invention, the NFκB inhibiting compound can be administered locally to the perispinal region of a spine or can be administered locally to the foraminal space, the epidural space or the intrathecal space of a spinal cord. These compounds can also be administered locally from an administration route selected from the group consisting of a catheter and drug pump, one or more local injections, polymer release, and combinations thereof.

The inhibitor of the present invention can be used to treat, sciatica and/or radicular pain and herniated disc.

The present invention encompasses dosing regimens. In one dosing regimen according to the present invention, the dosing regimen comprises an NFκB inhibiting compound selected from sulfasalazine or sulindac or combinations thereof and instructional information that directs the administration of the NFκB inhibiting compounds for the local treatment of pain.

In accordance with the present invention the NFκB inhibiting compounds can be administered locally to the perispinal region of a spine or locally to the foraminal space, the epidural space or the intrathecal space of a spinal cord. The NFκB inhibiting compounds can be administered locally from an administration route selected from the group consisting of a catheter and drug pump, one or more local injections, polymer release, and combinations thereof.

The present invention can include one or more of: (i) an administration form generally; (ii) an administration form comprising a catheter and drug pump, (iii) an administration form comprising one or more syringes for local injections, (iv) an administration form comprising compositions adapted for polymer release and (v) combinations thereof.

The present invention also includes compositions. In one embodiment of the compositions according to the present invention, the composition comprise an NFκB inhibiting compound selected from sulfasalazine or sulindac or combinations thereof wherein the one or more NFκB inhibiting compounds are directed to be administered locally for the treatment of pain as defined above.

### BRIEF DESCRIPTION OF THE FIGURES

FIG. 1 depicts a schematic representation of the NFκB activation pathway.

FIGS. 2 and 3 show the effect of the NFKB inhibitor sulfasalazine on pain sensitivity as measured by paw withdrawal latencies.

FIG. 4 shows the effect of the NFκB inhibitor sulfasalazine on pain sensitivity as measured in a mechanical allodynia paradigm.

FIGS. **5A-5B** show the effect of additional NFκB inhibitors on pain sensitivity measured by paw withdrawal latencies and in a mechanical allodynia paradigm.

FIG. 6 depicts the effect of low doses of sulindac on pain sensitivity as measured by paw withdrawal latencies

### DETAILED DESCRIPTION

While the sensation of pain can serve beneficial purposes, in many instances, such as neuropathic pain (which occurs in the absence of injury or infection) it does not and is highly undesirable. Problematic pain requiring treatment is believed to be caused at least in part due to local immune activation. The local immune activation is mediated largely by pro-inflammatory cytokines including interleukin-1 beta ("IL-1β'), tumor necrosis factor alpha ("TNFα") and interleukin-6 ("IL-6"). Sciatica provides one non-limiting example of pain that can be caused by local pro-inflammatory cytokine activity.

As the foregoing suggests, inhibiting the actions of pro-inflammatory cytokines can provide an effective strategy for treating pain. Inhibiting the immune system, however, is problematic as a general treatment because it leaves an individual vulnerable to infection and unable to repair tissue injuries effectively. Thus, treatments that systemically inhibit pro-inflammatory cytokines throughout the body are not appropriate except in the most extreme cases.

For a protein such as a pro-inflammatory cytokine to exert an effect, the cell that will use or secrete the protein must create it. Thus, one avenue to inhibit the local actions of pro-inflammatory cytokines is to inhibit intracellular mechanisms that lead to their production and release. The NFκB family is the primary group of transcription factors that plays an essential role in regulating the transcription of genes encoding pro-inflammatory cytokines (TNFα, IL-1β, IL-6), chemokines (IL-8, MIP1α), inducible effector enzymes (iNOS and COX-2), and other molecules. Thus, NFκB pathway inhibition is one attractive therapeutic strategy or the treatment of inflammatory and pain disorders. Effective NFκB pathway blockade can result in lower levels of an array of molecules including pro-inflammatory cytokines that contribute to inflammation and pain. However, because NFκB is also involved in normal cellular physiology such as mounting an effective immune response, systemic inhibition of the pathway may result in serious side effects. For example global inhibition of the NFκB pathway in adult animals can render them susceptible to opportunistic infections. Further, gene targeting studies in mice have shown that complete inactivation of nearly any member of the NFκB pathway (at least during development) results in significant immune system defects and/or embryonic lethality. For these reasons, minimizing systemic exposure of animals to NFκB inhibitory compounds is an important component to a safe therapeutic strategy for the treatment of pain.

The NFκB transcription factor family represents a group of structurally related and evolutionarily conserved proteins that includes five members in mammals: Rel (c-Rel), RelA (p65), RelB, NFκB1 (p50), and NFκB2 (p52). These molecules form functional transcription factors by complexing into hetero- or homodimers of the NFκB/Rel protein subunits. The most prevalent form of NFκB is a heterodimer of the p65 and p50 subunits.

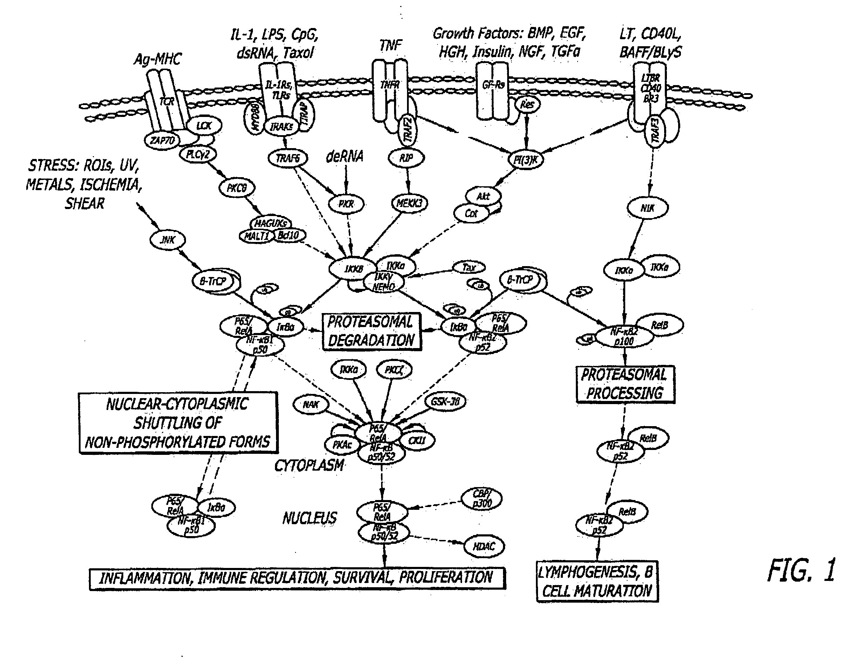

NFκB pathway activation (see FIG. 1) is regulated through a series of events. In unstimulated cells, NFκB is sequestered in the cytoplasm in an inactive form, bound to regulatory proteins called inhibitors of κB (IκB). A variety of stimuli including pro-inflammatory cytokines such as TNFα and IL-1β induce the phosphorylation of the IκB proteins (IκBα and IκBβ) at specific NH₂-terminal serine residues. The phosphorylated IκB proteins quickly become ubiquinated and degraded by the proteasome. The released NFκB proteins are then able to translocate to the cell nucleus and induce the transcription of a variety of genes containing their cognate DNA binding recognition sequences.

A key step in NFκB activation described in the preceding paragraph is the phosphorylation of the IκB proteins. This phosphorylation event is mediated by a specific protein complex known as IκB kinase (IKK). IKK is composed of two catalytic subunits IKKα and IKKβ, and a regulatory subunit named NFκB essential modulator (NEMO) or IKKγ. Cells deficient in either IKKα or IKKβ retain some inducible NFκB activity suggesting their distinct roles in NFκB pathway activation. Conversely, in cells lacking IKKγ, NFκB activation is completely blocked upon the induction of a variety of stimuli (including TNFα, IL-1, and lipopolysaccharide (LPS) exposure).

Regarding the use of NFκB inhibitors to treat pain, endoneural injections of an NFκB transcription factor decoy (at the site of peripheral injury) have been shown to significantly reduce thermal hyperalgesia in a rat model of neuropathic pain. In this model NFκB inhibition also results in lower levels of a variety of pro-inflammatory mediators (including TNFα, IL-1β, IL-6, IFN-γ, and iNOS). Sakaue *et al.,* (*Neuroreport.* 2001, 12(10):2079). Spinal administration of NFκB inhibitors (ODN decoys and pyrrolidine dithiocarbamate (PDTC)) have also been shown to significantly reduce mechanical allodynia and thermal hyperalgesia in the Complete Freund's Adjuvant (CFA) inflammatory pain model. Lee et al., (Euro J. Neurosci. 2004, 19:3375). Further, Tegeder et al., (J Neurosci. 2004, 24(7):1637) have reported that a specific IKK-β inhibitor (S1627) reduces hyperalgesia in inflammatory and neuropathic pain models (zymosan-induced paw inflammation) in rats. In addition, this inhibitor also reduces tactile allodynia in the chronic constriction injury model (CCI) of neuropathic pain. These studies demonstrate the efficacy of NFκB pathway blockade in the treatment of inflammatory and neuropathic pain.

Co-pending application publication number US2005/0095246A1 ("the '246 application") describes techniques to treat neurological disorders by attenuating the production of pro-inflammatory mediators. The'246 application describes the use of devices such as pumps/catheters and polymer-based drug depots for the local (peripheral, intrathecal, intraparenchymal) delivery of inhibitors of pro-inflammatory mediators (including members of the NFκB pathway; IKK-α, β, and γ) to treat inflammatory disorders. Embodiments described in the present application stem from these initial disclosures and also provide novel compounds to locally inhibit NFκB in the local treatment of pain through the local administration of these compounds.

### Examples

The behavioral rat animal model of chronic constriction injury ("CCI") was chosen to evaluate the efficacy of NFκB inhibitors as a pain treatment. This model may mimic pain associated with sciatica in humans. To induce CCI, each animal was anesthetized by intraperitoneal ("i.p.") injection of sodium pentobarbital at a dose of 60 mg/kg body weight. The animal's right common sciatic nerve was exposed and freed from adherent tissue at mid-thigh by separating the muscle (biceps femoris) by blunt dissection. Four loose ligatures were placed 1 mm apart, using chromic gut (4-0 absorbable suture, Jorgensen Laboratories Inc., Loveland, CO).

Animal behavioral testing for thermal hyperalgesia was conducted on Days -2 (baseline), 7, 14 and 21 after CCI. Animals were placed in the clear plastic chamber of a plantar analgesia instrument and allowed to acclimate to the environment for 15 minutes. After the acclimation period, a radiant (heat) beam source stimulus was applied to the CCI hind paw of each animal. The heat source device was set at an intensity of 50, and a maximum latency period of 15 seconds was set to prevent tissue damage according to the recommendations of the instrument manufacturer- If a paw withdrawal occurred within the 15 second period an automated control interrupted both the stimulus and timer, turning off the radiant beam and recording the latency of time to paw withdrawal. Data was analyzed using a one-way analysis of variance at each test day.

### Example 1

Animals were randomly assigned to treatment groups and administered control or test compounds as follows: animals received intraperitoneal (IP) injections of either vehicle (1 x Phosphate Buffered Saline; PBS) or the protein-based TNFα inhibitor, Enbrel^{®} as a positive control (3.0 mg/kg every 3 days; Immunex Corp., Seattle, WA), or daily subcutaneous (SC) injections of sulfasalazine, a small molecule inhibitor of NFκB at a dose of 5.0 or 50.0 mg/kg.

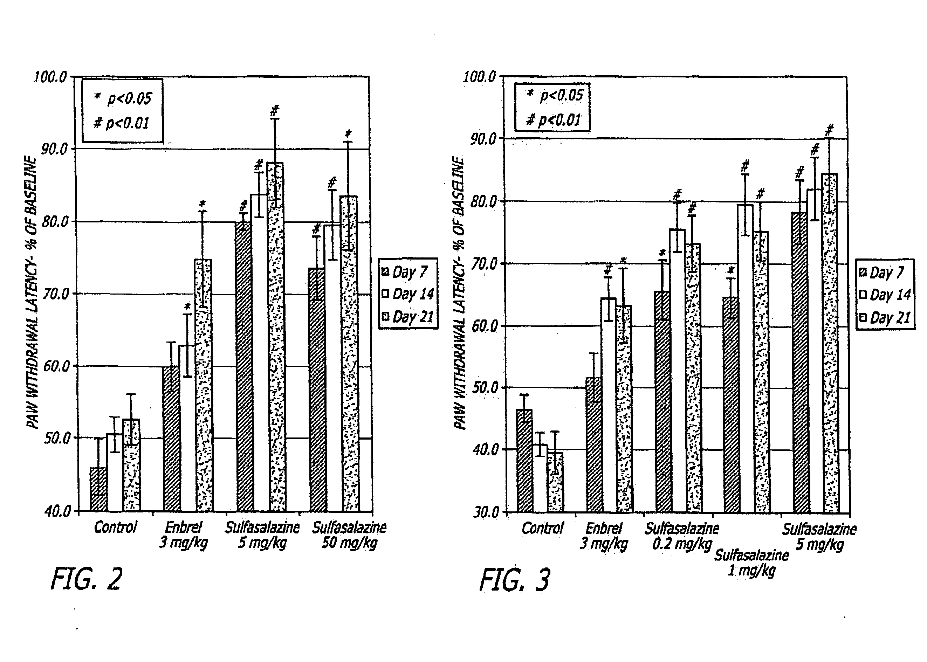

FIG. 2 demonstrates that the protein-based pro-inflammatory cytokine inhibitor Enbrel^{®} is effective to inhibit pain associated with CCI on all test days. NFκB inhibitors, however, were more effective at inhibiting pain associated with CCI on all test days. Specifically, animals that received vehicle showed mean paw withdrawal latencies of about 45%, 50% and 53% over baseline on test days 7, 14 and 21 respectively. Animals that received Enbrel^{®} showed mean paw withdrawal latencies of about 60%, 63% and 75% over baseline on test days 7, 14 and 21 respectively. Animals receiving 5 mg/kg sulfasalazine showed mean paw withdrawal latencies of about 80%, 83% and 87% over baseline while those receiving 50 mg/kg showed mean paw withdrawal latencies of about 74%, 79% and 83% over baseline on test days 7, 14 and 21 respectively. This data demonstrates that NFκB inhibition can provide an effective mechanism to decrease pain sensitivity.

### Example 2

In a subsequent study, additional lower doses of sulfasalazine were evaluated for their effectiveness as a pain treatment using the CCI model. Specifically, the same methods as described above were used. Sulfasalazine was administered at doses of 5.0; 1.0 or 0.2 mg/kg. As can be seen in FIG. **3**, control animals receiving vehicle showed paw withdrawal latencies averaging an increase of about 45%, 41% and 39% over baseline on test days 7, 14 and 21 respectively. Positive control animals receiving the protein-based TNFα inhibitor, Enbrel^{®} increased paw withdrawal latencies to about 51%, 63% and 62% over baseline on test days 7, 14 and 21 respectively. Again, however, all doses of sulfasalazine increased paw withdrawal latencies on all test days even further (to an average of between about 65% to about 85% over baseline measures on all test days), again suggesting that sulfasalazine and NFκB inhibition can provide an effective pain treatment. Indeed, this data suggests that sulfasalazine can provide a more effective pain treatment than protein-based inhibitors such as Enbrel^{®}.

### Example 3

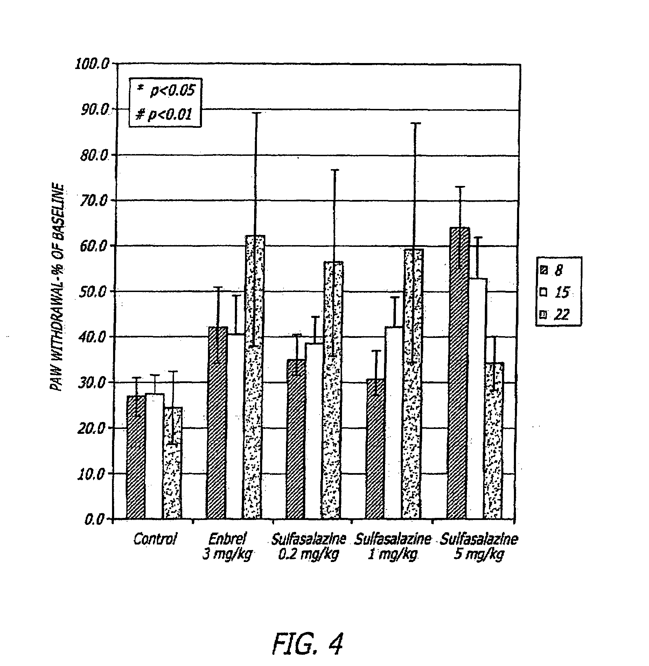

The ability of sulfasalazine to inhibit pain was also evaluated using a different behavioral measure following CCI, namely mechanical (or tactile) allodynia. In this study, mechanical allodynia was determined in reaction to probing with von Frey filaments (Stoelting, Wood Dale, IL). Mechanical sensitivity was measured on Days -1 (baseline), 8, 15 and 22 following CCI by determining the median 50% foot withdrawal threshold for von Frey filaments using the up-down method described in Chaplan et al. (J Neurosci Methods 1994; 54:55) which is incorporated by reference herein for its teachings regarding the up-down method. Rats were placed under a plastic cover (9 × 9 × 20 cm) on a metal mesh floor. The area tested was the middle glabrous area between the footpads of the plantar surface of the injured hind paw within the L4 innervation area. The plantar area was touched with a series of 9 von Frey hairs with approximately exponentially incremental bending forces (von Frey values: 3.61, 3.8, 4.0, 4.2, 4.41, 4.6, 4.8, 5.0, and 5.2; equivalent to: 0.41, 0.63, 1.0, 1.58, 2.51, 4.07, 6.31, 10, and 15.8 g). The von Frey hair was presented perpendicular to the plantar surface with sufficient force to cause slight bending and held for approximately 3 to 4 seconds. Abrupt withdrawal of the foot (paw flinching) was recorded as a response. Any rat showing a mechanical threshold of more than 3.24 g was eliminated from the study. As can be seen in FIG. **4**, Enbrel^{®} and sulfasalazine both decreased sensitivity in this paradigm when compared to vehicle, further suggesting that sulfasalazine and NFκB inhibition can provide an effective pain treatment when administered at appropriate dosages.

### Example 4

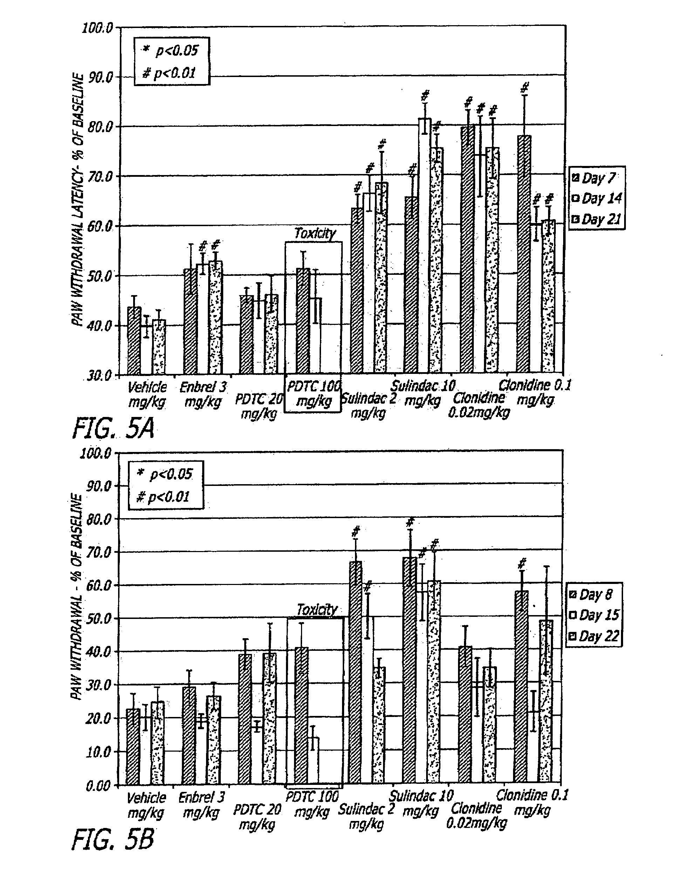

Next, the ability of other NFκB inhibitors to decrease pain sensitivity following CCI were evaluated. In this study, the previously described methods for inducing CCI and measuring thermal hyperalgesia and mechanical allodynia were followed except that animals received vehicle or 3 mg/kg Enbrel^{®} IP every 3 days, or daily IP injections of 20.0 or 100.0 mg/kg pyrollidinedithiocarbamate (PDTC); or 2.0 or 10.0 mg/kg sulindac; or daily SC injections of 0.02 or 0.1 mg/kg clonidine. As can be seen in FIG. **5A**, vehicle showed average paw withdrawal latencies of about 41% over baseline on all three test days. Enbrel^{®} increased paw withdrawal latencies to an average of about 51 % over baseline on all three test days. Animals receiving 2 mg/kg sulindac increased latencies to about 65% over baseline on all three test days while those receiving 10 mg/kg increased latencies to about 65%, 81% and 75% over baseline on test days 7, 41 and 21 respectively. Animals receiving 0.02 mg/kg clonidine showed an increase in paw withdrawal latencies over baseline of about 75%-79% on all three test days and those receiving 0.1 mg/kg clonidine showed an increase of about 78%, 60% and 61% over baseline on test days 7, 41 and 21 respectively. This data suggests that NFκB inhibitors reduce pain sensitivity further suggesting that NFκB inhibition can provide an effective pain treatment. Interestingly, in this study, while PDTC did increase paw withdrawal latencies over control levels, this compound was not as effective at reducing pain sensitivity as other NFκB inhibiting compounds. This result could be a function of dose or administration route. Indeed, animals receiving 100 mg/kg PDTC were removed from the study following the second day of testing due to drug toxicity.

In the behavioral test for mechanical allodynia, as can be seen in FIG. 5B, all NFκB inhibitors, (with the potential exception of PDTC), decreased pain sensitivity when compared to vehicle or Enbrel^{®}. Both doses of sulindac and the higher dose (0.1 mg/kg) of clonidine most significantly decreased pain sensitivity. These compounds decreased pain sensitivity on days 8, 15 and 22 respectively as follows: sulindac (2 mg/kg): about 66%, 50% and 35%; sulindac (10mg/kg): about 68%, 58% and 60%; clonidine (0.1 mg/kg): about 58%, 20% and 48%. Animals receiving vehicle or Enbrel^{®} showed increases between about 19% and 25%. Again, while PDTC showed some effect in decreasing pain sensitivity, the effect was not as strong as that seen with sulindac or clonidine. Further, while the low dose of clonidine decreased sensitivity, it did not show as strong as an effect in the mechanical allodynia test.

### Example 5

The effect of local, low doses of sulfasalzine on pain sensitivity was explored. Male Wistar rats were administered sulfasalazine at doses of 2.4 µg/day (0.72 µg/kg/day), 12.0 µg/day (3.6 µg/kg/day), or 60.0 µg/day (18 µg/kg/day); Etanercept (positive control) at a dose of 24.0 µg/day (7.2 µg/kg/day); or 30% DMSO in PBS (negative control) through a SC implanted Alzet pump as described above. Again, the previously described methods for CCi, thermal paw withdrawal latency measurements, and mechanical (or tactile) allodynia using von Frey filaments were followed.

Administration.of all test articles started on the day of surgery. Sulfasalazine was administered at doses of 2.4, 12.0, or 60.0 µg/day. Etanercept was administered at a dose of 24.0 µg/day. DMSO (30%) in PBS was administered as a control for sulfasalazine.

The behavioral data for thermal hyperalgesia are shown in Table 1 for sulfasalazine. The overall ANOVA indicates that all three doses of sulfasalazine (2.4, 12.0 or 60.0 µg/day) significantly increased paw withdrawal latency for thermal hyperalgesia when compared with DMSO [F(3, 24) = 4.80, p<0.05]. One-way ANOVA and Fisher LSD revealed that the effect was significant only on test Days 14 and 21 (Fisher LSD, p<0.05) but not on Day 7. The low dose, 2.4 µg/day, did not have significant effect when compared to DMSO on any of the testing days (Fisher LSD, n.s.).

**Table 1. Thermal Hyperalgesia Paw Withdrawal Latencies as a Percent of Baseline - Sulfasalazine**

| Treatment | | | Dose Level* | |
|---|---|---|---|---|
| Catheter/Pump Administration | | 2.4 µg/day | 12.0 µg/day | 60.0 µg/day |
| Day 7 | Mean | 79.1 | 80.6 | 83.8 |
| | SE | 3.2 | 2.5 | 6.0 |
| | N 7 | | 7 | 7 |
| Day 14 | Mean | 69.6 | 83.3 | 88.0 |
| | SE | 5.8 | 6.7 | 4.6 |
| | N 7 | | 7 | 7 |
| Day 21 | Mean | 77.1 | 79.1 | 85.2 |
| | SE | 2.3 | 3.1 | 5.3 |
| | N 7 | | 7 | 7 |

| | | | | |
|---|---|---|---|---|
| *Dose levels of 2.4, 12.0 and 60.0 µg/day correspond to infusion rates of 0.1, 0.5, and 2.5 µg/h, respectively. | | | | |

When comparing the three doses of sulfasalazine with etanercept, the overall ANOVA indicates that sulfasalazine produced a significant increase in latency on Day 7 (Day 7 t(12) = -2.50, p<0.05) and Day 21 (Day 21 t(12) = -2.3, p<0.05) but not on Day 14 (Day 14 t(12) = -0.30. n.s). The 12.0 µg/day t-test results show a significant increase in latency on Day 7 (Day 7 t(12) = -3.00, p<0.05) and Day 21 (Day 21 t(12) = -2.50, p<0.05) but not on Day 14 (Day 14 t(12) = -1.80, n.s.). The 60.0 µg/day t-test results show a significant increase in latency on all testing days: Day 7 t(12) = -2.40, p<0.05; Day 14 t(12) = -2.80, p<0.05; Day 21 t(12) = -2.80, p<0.05.

The data for mechanical allodynia are shown in Table 2. The overall ANOVA indicated that sulfasalazine treatment produced significant effects on mechanical allodynia when compared with DMSO [F(3, 24) = 3.50, p<0.05]. One-way ANOVA revealed that sulfasalazine produced an increase in mechanical thresholds when compared to DMSO on Day 22 [F(3. 24) = 4.30, p<0.05] but not on Days 8 [F(3, 24) = 2.90, n.s.] and 15 [F(3, 24) = 3.40, n.s.]. Fisher LSD results for all testing days indicated that only the high dose group showed a significant effect when compared to DMSO on Day 22 (Fisher LSD, p<0.05) but not on Days 8 and 15 (Fisher LSD, n.s.). The overall ANOVA indicated that sulfasalazine treatment did not have any significant effect on mechanical allodynia when compared to etanercept.

**Table 2. Von Frey Filament Test Paw Withdrawal Thresholds as a Percent of Baseline - Sulfasalazine**

| Treatment | | | Dose Level* | |
|---|---|---|---|---|
| Catheter/Pump Administration | | 2.4 µg/day | 12.0 µg/day | 60.0 µg/day |
| Day 8 | Mean | 47.6 | 43.9 | 68.6 |
| | SE | 6.8 | 14.9 | 6.3 |
| | N | 7 | 7 | 7 |
| Day 15 | Mean | 34.4 | 37.5 | 38.7 |
| | SE | 9.5 | 10.1 | 17.6 |
| | N | 7 | 7 | 7 |
| Day 22 | Mean | 46.3 | 47.1 | 84.3 |
| | SE | 10.7 | 6.4 | 18.5 |
| | N | 7 | 7 | 7 |

| | | | | |
|---|---|---|---|---|
| *Dose levels of 2.4, 12.0 and 60.0 µg/day correspond to infusion rates of 0.1, 0.5, and 2.5 µg/h, respectively. | | | | |

No overt side effects or adverse reactions occurred In any of the animals treated with sulfasalazine, etanercept, or DMSO over the duration of this 22-day study.

The data presented in this study show the effectiveness of the test articles in the rat CCI model and demonstrate that local delivery of low dosages of NFκB inhibitors have therapeutic potential in the treatment of neuropathic pain.

### Example 6

Following the previous experiment, the effect of local, low doses of sulindac on pain sensitivity was explored. Male Wistar rats were administered sulindac at doses of 0.0016 µg/hour (0.01152 µg/kg/day), 0.008 µg/hour (0.0576 µg/kg/day), or 0.04 µg/hour (0.288 µg/kg/day); clonidine at a dose of 0.0004 µg/hour (0.00288 µg/kg/day), 0.002 µg/hour (0.0144 µg/kg/day), or 0.01 µg/hour (0.072 µg/kg/day); or vehicle through a SC implanted Alzet pump. Again, the previously described methods for CCI and thermal paw withdrawal latency measurements.

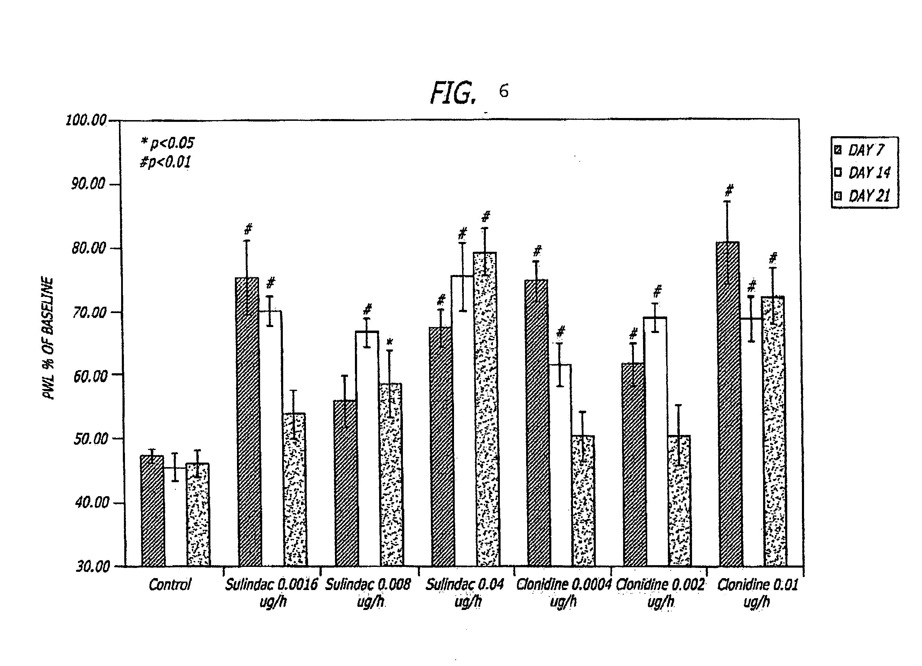

As can be seen in FIG. 6, sulindac at 0.04 µg/hour provided significant increases In paw withdrawal latencies following thermal stimulus on all three test days compared to vehicle and provided increased paw withdrawal latencies following thermal stimulus on Days 14 and 21 compared to 0.01 µg/hour of clonidine.

This low dosing of sulindac can substantially reduce the systemic exposure of the drug without compromising the efficacy of the treatment.

### Example 7

In the rat CCI model using the same behavioral tests described above, efficacy (p<0.05 compared to vehicle) has been demonstrated with suffasalazine for doses as low as 0.05 µg/hr (12 µg/day).

### Example 8

Drug depots in the form of pellets containing either 20% sulindac or 25% sulindac were made using the process described below.

Approximately 50 grams of 85/15 poly(D,L-lactide-co-glycolide) (PLGA) (Lakeshore Biomaterials, Birmingham, AL) with IV of 0.75 dUg and molecular weight of 117 kDa, was placed in a polypropylene beaker and cooled with liquid nitrogen (approximately 200 mL) for 10 minutes. The polymer was then ground into fine particles of approximately 80 microns average diameter using a Ultra Centrifugal Mill ZM 200 (Retsch GmbH & Co., Haan, Germany). The ground polymer particles were collected and placed in 10 cm aluminum weigh pans. The pans were placed in a vacuum oven at 35°C under vacuum for 24 hours to remove any condensation resulting from the grinding process.

The polymer was weighed into an aluminum weigh pan using an analytical balance. Sulindac (spectrum Chemical, Gardena, CA) was then added to the ground polymer: The mixture was stirred using a spatula until the polymer and drug appeared to be uniformly mixed. Polyethylene glycol methyl ether (mPEG) (MW 550, Sigma-Aldrich, St. Louis, MO) was then added to the drug and polymer mixture. The components were mixed using a spatula, until the mixture appeared homogeneous.

For the 20% Sulindac formulation the following amounts were used: 3.5 grams PLGA; 1.10 gram sulindac; 0.55 grams mPEG. For the 25% sulindac formulation the following amounts were used: 3.5 grams PLGA; 1.35 grams sulindac; 0.55 grams mPEG.

Each mixture was then loaded into a HAAKE MiniLab Rheomex extruder (Model CTW5, Thermo Electron Corp., Waltham, MA), and extruded through a die of 0.75 mm diameter (temperature 120°C, 25 rpm). The resulting polymeric strand was then cut into cylindrical pellets approximately 0.75 mm in length (aspect ratio = 1). The cut pellets were stored in a sealed glass vial, which had been purged with dry nitrogen, until needed.

Approximately 25 mg of the pellets of each concentration of sulindac were weighed into each of 3 vials containing 10 mL of PBS, (pH 7.4). The vials were sealed and placed in a Model C24 incubator/shaker (New Brunswick Scientific Co, Edison, NJ) set at 37°C and agitated at approximately 70 RPMs. At specific time points, the elution buffer was removed and analyzed for drug using a UV/vis spectrophotometer at 328 nm (Model: Lambda 850, Perkin Elmer, Waltham, MA). The sample vials were replenished with fresh PBS and returned to the incubator/shaker until the next timepoint. The cumulative drug released was plotted as a percentage of the initial drug payload.

The 25% sulindac pellets had approximately 30% of the drug eluted by day 20, approximately 40% of the drug eluted by day 40, approximately 45% eluted by day 60, and approximately 60% eluted by day 60. The 20% sulindac pellets had approximately 20% of the drug eluted by day 20, approximately 25% of the drug eluted by day 40, less than 30% eluted by day 60, and approximatety 45% eluted by day 80.

The disclosed invention describes the use of a therapeutic agent to block activation of the NFκB signaling, pathway to alleviate pain. Pain is likely reduced by these inhibitors through their effect in reducing levels of pro-inflammatory cytokines and other molecules involved in the inflammation response. The therapeutic agent may be a small molecule inhibitor of NFκB pathway activation or other effective NFκB inhibitors. Non-limiting examples of potential therapeutic agents for use in accordance with the present invention can include anti-oxidants that have been shown to inhibit NFκB, proteasome and protease Inhibitors that inhibit NFκB, and IκBo phosphorylation and/or degradation inhibitors. Example of such compounds include, without limitation, α-lipoic acid, α-tocopherol, allicin, 2-amino-1-methyl-6-phenylimidazo[4,5-b]pyridine, anetholdithiolthione, apocynin, 5,6,3,5-tetramethoxy 7,4'-hydroxyflavone, astaxanthin, benidipine, bis-eugenol, brugulera gymnorrhiza compounds, butylated hydroxyanisole, cepharanthine, caffeic acid phenethyl ester, camosol, β-carotene, carvedilol, catechol derivatives, chlorogenic and, cocoa polyphenols, curcumin, dehydroepiandresterone and dehydroepiandrosterone sulfate, dibenzylbutyrolactone lignans, diethyldithlocarbamate, diferoxamine, dihydroisoeugenol, dihydrolipoic acid, dilazep + fenofibric acid, dimethyldithiocarbamates, dimethylsulfoxide, disulfiram, ebselen, edaravone, epc-k1, epigallocatechin-3-gallate, ergothioneine, ethylene glycol tetraacetic acid, flavonoids (crataegus; boerhaavia diffusa root; xanthohumol), γ-glutamylcysteine synthetase, ganoderma lucidum polysaccharides, garcinol, ginkgo biloba extract, hematein, 23-hydroxyursolic acid, iron tetrakis, isovitexin, kangen-karyu extract, I-cysteine, lacidipine, lazaroids, lupeol, magnolol, maltol, manganese superoxide dismutase, extract of the stem bark of mangifera indica I, melatonin, mulberry anthocyanins, n-acetyl-I-cysteine, nacyselyn, nordihydroguaiaritic acid, ochnaflavone, orthophenanthroline, hydroquinone, tert-butyl hydroquinone, phenylarsine oxide, phyllanthus urinaria, pyrrolinedithiocarbamate, quercetin (low concentrations), redox factor 1, rotenone, roxithromycin, s-allyl-cysteine, sauchinone, spironolactone, strawberry extracts, taxifolin, tempol, tepoxaline, vitamin C, vitamin B6, vitamin E derivatives, α-torphryl succinate, α-torphryl acetate, 2,2,5,7,8-pentamethyl-6-hydroxychromane, yakuchinone α and β, n-acetyl-leucinyl-leucynil-norleucynal, n-acetyl-leucinyl-leucynil-methional, carbobenzoxyl-leucinyl-leucynil-norvalinal, carbobenzoxyl-leucinyl-leucynil-leucynal, lactacystine, β-lactone, boronic acid peptide, ubiquitin ligase inhibitors, bortezomib, salinosporamide α, cyclosporin α, tacrolimus, deoxyspergualin, 15 deoxyspergualin, analogs of 15-deoxyspergualin, n-acetyl-dl-phenylalanine-β-naphthylester, n-benzoyl I-tyrosine-ethylester, 3,4-dichloroisocoumarin, diisopropyl fluorophosphate, n-α-tosyl-I-phenylalanine chloromethyl ketone, n-α-tosyl-I-lysine chloromethyl ketone, desloratad,ine, salmeterol, fluticasone propionate, protein-bound polysaccharide from basidiomycetes, calagualine, golli bg21, npm-alk oncoprotein, ly29, ly30, ly294002, evodiamine, rituximab, kinase suppressor of ras, pefabloc, rocaglamides, betaine, tnap, geldanamycin, grape seed proanthocyanidins, pomegranate fruit extract, tetrandine, 4(2'-aminoethyl)amino-1,8-dimethylimidazo(1,2-α) quinoxaline, 2-amino-3-cyano-4-aryl-6-(2-hydroxy-phenyl)pyridine derivatives, acrolein, anandamide, as602868, cobrotoxin, dihydroxyphenylethanol, herbimycin α, inhibitor 22, isorhapontigenin, manumycin α, mlb120, nitric oxide, nitric oxide donating aspirin, thienopyridine, acetyl-boswellic acids, β-carboline, cyl-19s, cyl-26z, synthetic α-methylene-y-butyrolactone derivatives, 2-amino-6-[2-(cyclopropylmethoxy)-6-hydroxyphenyl]-4-piperidin-4-yl nicotinonitrile, plant compound α, flavopiridol, cyclopentones, jesterone dimmer, ps-1145, 2-[(aminocarbonyl)amino]-5-acetylenyl-3-thiophenecarboxamides, 1' acetoxychavicol acetate, apigenin, cardamomin, synthetic triterpenoid, chs 828 (anticancer drug), diosgenin, furonaphthoquinone, guggulsterone, heparin-binding epidermal growth factor-like growth factor, falcarindol, hepatocyte growth factor, honokiol, hypoestoxide, γ-mangostin, garcinone β, kahweol, kava derivatives, ml120b, mx781 (retinoid antagonist), n-acetylcysteine, nitrosylcobalamin (vitamin B12 analog), non-steroidal anti-inflammatory drugs (NSAIDs), hepatits c virus ns5b, pan1 (aka nalp2 or pypaf2), n-(4-hydroxyphenyl) retinamide, sulforaphane, phenylisothiocyanate, survanta, piceatannol, 5-hydroxy-2-methyl-1,4-naphthoquinone, pten (tumor suppressor), theaflavin, tilianin, zerumbone, silibinin, sulfasalazine, sulfasalazine analogs, rosmarinic acid, staurosporine, γ tocotrienol, wedelolactone, betulinic acid, ursolic acid, thalidomide, interleukin-10, mollusum contagiosum virus mc159 protein, monochloramine, glycine chloramine, anethole, anti-thrombin III, artemisia vestita, aspirin, sodium salicylate, azidothymidine, baoganning, e3((4-methylphenyl)-sulfonyl)-2-propenenitrile, e3((4-t-butylphenyl)-sulfonyl)-2-propenenitrile, benzyl isothiocyanate, cyanidin 3-o-glucoside, cyanidin 3-o-(2(g)-xylosylrutinoside, cyanidin 3-o-rutinoside, buddlejasaponin IV, cacospongionolide β, carbon monoxide, carboplatin, cardamonin, chorionic gonadotropin, cycloepoxydon, 1-hydroxy-2-hydroxymethyl-3-pent-1-enylbenzene, decursin, dexanabinol, digitoxin, diterpenes (synthetic), docosahexaenoic acid, extensively oxidized low density lipoprotein, 4-hydroxynonenal, fragile histidine triad protein, gabexate mesilate, [6]-gingerol, casparol, imatanib, glossogyne tenuifolia, ibuprofen, indirubin-3'-oxime, interferon-α, licorice extracts, methotrexate, nafamostat mesilate, oleandrin, omega 3 fatty acids, panduratin α, petrosaspongiolide m, pinosylvin, plagius flosculosus extract polyacetylene spiroketal, phytic acid, prostaglandin α1, 20(s)protopanaxatriol, rengyolone, rottlerin, saikosaponin-d, saline (low Na⁺ istonic), salvia miltiorrhizae water-soluble extract, pseudochelerythrine, 13-methyl-[1,3]-benzodioxolo-[5,6-c]-1,3-dioxolo-4,5 phenanthridinium), scoparone, silymarin, socs1, statins, sulindac, thi 52 (1-naphthylethyl-6,7-dihydroxy-1,2,3,4-tetrahydroisoquinoline), 1,2,4-thiadiazolidine derivatives, vesnarinone, xanthoangelol d, yc-1, yopj, acetaminophen, activated protein c, alachlor, α-melanocyte-stimulating hormone, amentoflavone, artemisia capillaris thunb extract, artemisia iwayomogi extract, I-ascorbic acid, antrodia camphorate, aucubin, baicalein, β-lapachone, blackberry extract, buchang-tang, capsaicin, catalposide, core protein of hepatitis c virus, cyclolinteinone, diamide, dihydroarteanniun, dobutamine, e-73 (cycloheximide analog), ecabet sodium, emodin, ephedrae herba, equol, erbstatin, estrogen, ethacrynic acid, fosfomycin, fungal gliotoxin, gamisanghyulyunbueum, genistein, genipin, glabridin, glimepiride, glucosamine sulfate, glutamine, gumiganghwaltang, heat shock protein-70, hypochlorite, interleukin-13, isomallotochromanol, isomallotochromene, vaccinia virus protein, kochia scoparia fruit, leflunomide metabolite, losartin, 5'-methylthioadenosine, momordin I, morinda officinalis extract, murr1 gene product, neurofibromatosis-2 protein, u0126, penetratin, pervanadate, β-phenylethyl and 8-methylsulphinyloctyl isothiocyanates, phenytoin, platycodin saponins, polymyxin β, poncirus trifoliata fruit extract, probiotics, pituitary adenylate cyclase-activating polypeptide, prostaglandin 15-deoxy-delta(12,14)-pgj(2), resiniferatoxin, sabaeksan, saccharomyces boulardii anti-inflammatory factor, sesquiterpene lactones (parthenolide; ergolide; guaianolides), st2 (interleukin-1-like receptor secreted form), thiopental, tipifarnib, titanium, tnp-470, stinging nettle (urtica dioica) plant extracts, trichomomas vaginalis infection, triglyceride-rich lipoproteins, ursodeoxycholic acid, xanthium strumarium I, vasoactive intestinal peptide, HIV-1 vpu protein, epoxyquinone α monomer, ro106-9920, conophylline, mol 294, perrilyl alcohol, mast205, rhein, 15-deoxy-prostaglandin j(2), antrodia camphorata extract, β-amyloid protein, surfactant protein α, dq 65-79 (aa 65-79 of the α helix of the alpha-chain of the class II HLA molecule dqa03011), c5a, glucocorticoids (dexamethasone, prednisone, methylprednisolone), interleukin-10, interleukin-11, α-pinene, vitamin D, fox1j, dioxin, agastache rugosa leaf extract, alginic acid, astragaloside iv, atorvastatin, blue honeysuckle extract, n(1)-benzyl-4-methylbenzene-1,2-diamine, buthus martensi karsch extract, canine distemper virus protein, carbaryl, celastrol, chiisanoside, dehydroxymethylepoxyquinomicin, dipyridamole, diltiazem, eriocalyxin β, estrogen enhanced transcript, gangliosides, glucorticoid-induced leucine zipper protein, harpagophytum procumbens extracts, heat shock protein 72, hirsutenone, indole-3-carbinol, jm34 (benzamide derivative), 6-hydroxy-7-methoxychroman-2-carboxylic acid phenylamide, leptomycin β, levamisole, 2-(4-morpholynl) ethyl butyrate hydrochloride, nls cell permeable peptides, 2',8"-biapigenin, nucling, o,o'-bismyristoyl thiamine disulfide, oregonin, 1,2,3,4,6-penta-o-galloyl-β-d-glucose, platycodi radix extract, phallacidin, piperine, pitavastatin, pn-50, rela peptides (p1 and p6), retinoic acid receptor-related orphan receptor-α, rhubarb aqueous extract, rolipram, salvia miltiorrhoza bunge extract, sc236 (a selective cox-2 inhibitor), selenomethionine, sophorae radix extract, sopoongsan, sphondin, younggaechulgam-tang, zud protein, zas3 protein, clarithromycin, fluvastatin, leflunomide, oxidized 1-palmitoyl-2-arachidonoyl-sn-glycero-3-phosphorylcholine, serratamolide, moxifloxacin, sorbus commixta cortex, cantharidin, cornus officinalis extract, neomycin, omapatrilat, enalapril, cgs 25462, onconase, paeoniflorin, rapamycin, sargassum hemiphyllum methanol extract, shenfu, tripterygium polyglycosides, triflusal, hepatoma protein, andrographolide, melittin, 1'-acetoxychavicol acetate, 2-acetylaminofluorene, actinodaphine, adiponectin, nicotinamide, 3-aminobenzamide, 7-amino-4-methylcoumarin, amrinone, angiopoietin-1, anthocyanins, sequiterpene lactones, artemisinin, atrial natriuretic peptide, atrovastat, avra protein, baicalein, benfotiamine, β-catenin, biliverdin, bisphenol α, bovine serum albumin, brazilian, bromelain, calcium/calmodulin-dependent kinase kinase, calcitriol, campthothecin, sutherlandia frutescens, caprofin, capsiate, carbocisteine, cat's claw bark, maca, celecoxib, germcitabine, cheongyeolsaseuptang, chitosan, ciclosporin, cinnamaldehyde, 2-methoxycinnamaldehyde, 2- hydroxycinnamaldehyde, guaianolide 8-deoxylactucin, chlorophyllin, chondrotin sulfate proteoglycan degradation product, clarithromycin, cloricromene, commerical peritoneal dialysis solution, compound K, 6-hydroxy-7-methoxychroman-2-carboxylic acid phenylamide, cryptotanshinone, cyanoguanidine, cytochalasin d, da-9201 (from black rice), danshenshu, decoy oligonucleotides, diarylheptanoid 7-(4'-hydroxy-3'-methoxyphenylyl-phenylhept-4-en-3-one, α-difluoromethylornithine, dim/13c, diterpenoids from isodon rubescens or liverwort jungermannia, 4,10-dichloropyrido[5,6:4,5]thieno[3,2- d':3,2- d]-1, 2, 3-ditriazine, e3330, entkaurane diterpenoids, epinastine hydrochloride, epoxyquinol α, erythromycin, evans blue, fenoldopam, fexofenadine hydrochloride, fibrates, fk778, flunixin meglumine, flurbiprofen, fomes fomentarius methanol extracts, fucoidan, glycoprotein-120, gallic acid, ganoderma lucidum, homeobox protein, geranylgeraniol, ghrelin, ginkgolide β, glycyrrhizin, halofuginone, helenalin, herbal compound 861, HIV-1 resistance factor, hydroxyethyl starch, hydroxyethylpuerarin, hypercapnic acidosis, hypericin, interleukin 4, IκB-like proteins, imd-0354, insulin-like growth factor binding protein-3, jsh-21 (n1-benzyl-4-methylbenzene-1,2-diamine), kamebakaurin, kaposi's sarcoma-associated herpesvirus k1 protein, ketamine, kt-90 (morphine synthetic derivative), linoleic acid, lithospermi radix, lovastatin, macrolide antibiotics, mercaptopyrazine, 2-methoxyestradiol, 6 (methylsulfinyl)hexyl isothiocyanate, metals (chromium, cadmium, gold, lead, mercury, zinc, arsenic), mevinolin, monomethylfumarate, moxifloxacin, myricetin, myxoma virus mnf, ndpp1, n-ethyl-maleimide, naringen, nicorandil, nicotine, nilvadipine, nitrosoglutathione, extracts of ochna macrocalyx bark, leucine-rich effector proteins of salmonella & shigella, omega-3 fatty acids oridonin 1,2,3,4,6-penta-o-galloyl-beta-d-glucose, interferon inducible protein, p21 (recombinant), peptide nucleic acid-DNA decoys, pentoxifylline (1-(5'-oxohexyl) 3,7-dimetylxanthine, peptide yy, pepluanone, perindopril, 6(5h)-phenanthridinone and benzamide, phenyl-n-tert-butylnitrone, phyllanthus amarus extracts, protein inhibitor of activatated stat1, pioglitazone, pirfenidone, polyozellin, prenylbisabolane 3, pro-opiomelanocortin, prostaglandin e2, protein-bound polysaccharide, pypaf1 protein, pyridine n-oxide derivatives, pyrithione, quinadril, quinic acid, raf kinase inhibitor protein, rapamycin, raloxifene, raxofelast, rebamipide, rhus verniciflua stokes fruits 36 kda glycoprotein, ribavirin, rifamides, ritonavir, rosiglitazone, sanggenon c. santonin diacetoxy acetal derivative, secretory leucoprotease inhibitor, n-(p-coumaroyl) serotonin, sesamin, simvastatin, sinomenine, sirt1 deacetylase overexpression, siva-1. sm-7368, solana nigrum I, 150 kda glycoprotein, sun c8079, tanacetum larvatum extract, tansinones, taurine + niacine, thiazolidinedione mcc-555, trichostatin α, triclosan plus cetylpyridinium chloride, triptolide, tyrphostin ag-126, uteroglobin, vascular endothelial growth factor, verapamil, withaferin α, 5,7-dihydroxy-8-methoxyflavone, xylitol, yan-gan-wan, yin-chen-hao, yucca schidigera extract, amp-activated protein kinase, apc0576, artemisia sylvatica, bsasm, bifodobacteria, bupleurum fruticosum phenylpropanoids, ebv protein, chromene derivatives, dehydroevodiamine, 4'-demethyl-6-methoxypodophyllotoxin, ethyl 2-[(3-methyl-2,5-dioxo(3-pyrrolinyl))amino]-4-(trifluoromethyl) pyrimidine-5-carboxylate, cycloprodigiosin hycrochloride, dimethylfumarate, fructus benincasae recens extract, glucocorticoids (dexametasone, prednisone, methylprednisolone), gypenoside xlix, histidine, HIV-1 protease inhibitors (nelfinavir, ritonavir, or saquinavir), 4-methyl-N1-(3-phenyl-propyl)-benzene-1,2-diamine, kwei ling ko, ligusticum chuanxiong hort root, nobiletin, NFκB repression factors, phenethylisothiocyanate, 4-phenylcoumarins, phomol, pias3, pranlukast, psychosine, quinazolines, resveratrol, ro31-8220, saucerneol d and saucerneol e, sb203580, tranilast, 3,4,5-trimethoxy-4'-fluorochalcone, uncaria tomentosum plant extract, mesalamine, mesuol, pertussis toxin binding protein, 9-aminoacridine derivatives (including the antimalaria drug quinacrine), adenosine and cyclic amp, 17-allylamino-17-demethoxygeldanamycin, 6-aminoquinazoline derivatives, luteolin, manassantins α and β, paromyxovirus sh gene products, qingkailing, shuanghuanglian, smilax bockii warb extract, tetracyclic a, tetrathiomolybdate, trilinolein, troglitazone, witheringia solanacea leaf extracts, wortmannin, α-zearalenol, antithrombin, rifampicin, and mangiferin (see http://people.bu.edu/gilmore/nf-kb/inhibitors/index.html which is incorporated by reference herein for a list of potential inhibitors). The presently disclosed invention can be especially beneficial because pain patients treated with protein-based cytokine inhibitors (for example and without limitation, etanercept or infliximab) often have immune responses directed against the recombinant (therapeutic) proteins. In the present invention, it is unlikely that there will be a significant immune response against a small molecule therapeutic compound.

The methods and NFκB inhibitors of the present inventions are useful for treating pain in mammals including, but not limited to, horses, dogs, cat, cattle, sheep, humans, non-human primates, rodents, rabbits, and other mammals in need of treatment for pain.

The present invention can be used to treat a variety of conditions related to NFκB activation and pro-inflammatory cytokine responses. For example, embodiments according to the present invention could be used to contribute to the treatment of without limitation, osteoarthritis, alkylosing spondylitis, psoriasis, rheumatoid arthritis (RA), sepsis and degenerative disc disease. Further, it may be beneficial to coat implantable medical devices such as, without limitation, stents and stent graft with small molecule NFκB pathway inhibitors.

In one embodiment according to the present invention, the therapeutic agents described herein are delivered locally in order to minimize undesirable side effects associated with systemic delivery of the immunosuppressive agents. When delivered to local sites containing cells that have a responsive NFκB pathway, the therapeutic agents can be delivered through a device consisting of an infusion pump and a catheter. Local sites of delivery can include, but are not limited to the nerve root, the dorsal root ganglion (DRG), and focal sites of inflammation (containing infiltrating inflammatory cells). The distal, delivery end of the catheter can be surgically positioned in the tissue in close proximity to the targeted site (nerve root, DRG, etc). Alternatively, the distal end of the catheter may be positioned to deliver the therapeutic compound into the intrathecal space of the spinal cord. For acute therapeutic compound delivery, the proximal end of the catheter could remain outside of the patient's body and be attached to an external, refillable pump. For chronic administration of the compound, the proximal end of the catheter could be attached to a pump implanted subcutaneously within a patient. In this case, the pump would be able to be periodically refilled using transcutaneous syringe injection.

The NFκB inhibitors can be locally delivered by catheter and drug pump systems, delivered by direct local injection or through the use of polymers and/or drug-eluting stents as described in co-pending U.S. Patent Application No. 10/972,157 which is incorporated by reference herein. In one embodiment, a "controlled administration system" including a direct and local administration system can be used. A controlled administration system can be a depot or a pump system, such as, without limitation, an osmotic pump or an infusion pump. An infusion pump can be implantable and can be, without limitation, a programmable pump, a fixed rate pump, and the like. A catheter can be operably connected to the pump and configured to deliver agents of the present invention to a target tissue region of a subject. A controlled administration system can be a pharmaceutical depot (a pharmaceutical delivery composition) such as, without limitation, a capsule, a microsphere, a particle, a gel, a coating, a matrix, a wafer, a pill, pellet, fiber, and the like. A depot can comprise a biopolymer. The biopolymer can be a sustained-release biopolymer. The depot can be deposited at or near, generally in close proximity, to a target site via a needle or catheter. Embodiments of the present invention also can be delivered through the use of liposomes, polyethyleneimine, by iontophoresis, or by incorporation into other vehicles, such as biodegradable or non-biodegradable nanocapsules. The delivery technology (drug pump or polymer formulations) can also be useful for the delivery of small molecules directed against other gene targets for other clinical indications.

Natural or synthetic biocompatible biodegradable biopolymers can be used to make the pharmaceutical depots of the present invention. Natural polymers include, but are not limited to, proteins such as albumin, collagen, gelatin synthetic poly(aminoacids), and prolamines; glycosaminoglycans, such as hyaluronic acid and heparin; polysaccharides, such as alginates, chitosan, starch, and dextans; and other naturally occurring or chemically modified biodegrable polymers. Synthetic biocompatible biodegradable materials include, but are not limited to, poly(lactide-co-glycolide) (PLGA), polylactide (PLA), polyglycolide (PG), polyhydroxybutyric acid, poly(trimethylene carbonate), polycaprolactone (PCL), polyvalerolactone, poly (alpha-hydroxy acids), poly(lactones), poly (amino-acids), poly(anhydrides), polyketals poly(arylates), poly(orthoesters), poly(orthocarbonates), poly(phosphoesters), poly(ester-co-amide), poly(lactide-co-urethane, polyethylene glycol (PEG), polyvinyl alcohol (PVA), PVA-g-PLGA, PEGT-PBT copolymer (polyactive), methacrylates, poly(N-isopropylacrylamide), PEO-PPO-PEO (pluronics), PEO-PPO-PAA copolymers, and PLGA-PEO-PLGA blends and copolymers thereof and any combinations thereof. As an example, poly(d,l-lactic-co-glycolic acid) is commercially available from Alkermes of Cambridge, MA. Suitable Alkermes products include Medisorb® 50:50 DL, 65:35 DL, 75:25 DL, 85:15 DL and poly(d,l-lactic acid) (d,l-PLA), where the product's mole percent composition of lactide and glycolide are given. For example, 75:25 DL have the mole percent ratios of 75% lactide and 25% glycolide.

Biopolymers may be prepared by the procedure set forth in U.S. Pat. No. 4,293,539 (Ludwig, et al.), the disclosure of which is hereby incorporated by reference in its entirety. Ludwig prepares such copolymers by condensation of lactic acid and glycolic acid in the presence of a readily removable polymerization catalyst (e.g., a strong acid ionexchange resin such as Dowex HCR-W2-H).

Microparticles can be made by solvent evaporation, phase separation, fluidized bed coating or combinations thereof. With solvent evaporation, a NFκB inhibiting compound, if soluble in organic solvents, may be entrapped in the biopolymer by dissolving the biopolymer in a volatile organic solvent, adding a NFκB inhibiting compound to the organic phase, emulsifying the organic phase in water which a surfactant or polymer such as polyvinyl alcohol, and finally removing the solvent under vacuum to form discrete, hardened monolithic micro-particles.

Phase separation procedures entrap water-soluble NFκB inhibiting compounds in the biopolymer to prepare micro-particles. Phase separation involves coacervation of a biopolymer. By addition of a nonsolvent, such as silicone oil, the biopolymer is then extracted from an organic solvent.

Alternatively, the micro-particles may be prepared by the process of Ramstack et al., 1995, described in published international patent application WO 95/13799, the disclosure of which is incorporated herein in its entirety. The Ramstack et al. process essentially provides for a first phase, including an active agent and a polymer, and a second phase, that are pumped through a static mixer into a quench liquid to form micro-particles containing the active agent. The first and second phases can optionally be substantially immiscible and the second phase is preferably free from solvents for the polymer and the active agent and includes an aqueous solution of an emulsifier.

In a fluidized bed coating, the drug is dissolved in an organic solvent along with the polymer. The solution is then processed, *e.g*., through a Wurster air suspension coating apparatus to form the final microcapsule product.

When the biopolymer and a NFκB inhibiting compound are mixed together, the biopolymer incorporates the NFκB inhibiting compound into pharmaceutical depot for possible sustained release of the drug at a target area within the body. The pharmaceutical depot may degrade in vivo over a period of less than about two years, where at least 50% of the drug depot dissolves anywhere from about 3 months to within about a year.

When a NFκB inhibiting compound is admixed with a biodegradable polymer for a controlled release into or near the site of a patient's pain, useful loadings of the NFκB inhibiting compound are from about 0.1% to about 99% (w/w) of the polymer, from about 1% to about 80%, from about 1% to about 50%, from about 1% to about 30% (w/w) of the polymer.

Dosages and desired drug concentrations of NFκB inhibiting compounds of the present invention may vary depending on the particular use envisioned. The determination of the appropriate dosage or route of administration is well within the skill of an ordinary physician. Animal experiments provide reliable guidance for the determination of effective doses for human therapy. Interspecies scaling of effective doses can be performed following the principles laid down by Mardenti, J. and Chappell, W. "The use of interspecies scaling in toxicokinetics" In Toxicokinetics and New Drug Development, Yacobi et al, Eds., Pergamon Press, New York 1989, pp. 42-96. The term "therapeutically effective" amount as used herein refers to the amount needed to perform the particular treatment such as, for example, treatment of pain. "Treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

In an embodiment of the present invention, the dosing rate of the NFκB inhibitors is not to exceed 100 µg/kg/day, 90 µg/kg/day, 80 µg/kg/day, 70 µg/kg/day, 60 µg/kg/day, 50 µg/kg/day, 40 µg/kg/day, 30 µg/kg/day, 20 µg/kg/day, or 10 µg/kg/day. These ranges are understood to include every integer between 100 and 10.

In one embodiment of the present invention, the NFκB inhibitor is sulfasalazine and the dosage is from approximately 0.005 to approximately 100 µg/kg/day. Additional dosages of sulfasalazine can include from approximately 0.005 to approximately 95 µg/kg/day; approximately 0.005 to approximately 90 µg/kg/day; approximately 0.005 to approximately 85 µg/kg/day; approximately 0.005 to approximately 80 µg/kg/day; approximately 0.005 to approximately 75 µg/kg/day; approximately 0.01 to approximately 70 µg/kg/day; approximately 0.01 to approximately 65 µg/kg/day; approximately 0.01 to approximately 60 µg/kg/day; approximately 0.01 to approximately 55 µg/kg/day; approximately 0.01 to approximately 50 µg/kg/day; approximately 0.01 to approximately 45 µg/kg/day; approximately 0.01 to approximately 40 µg/kg/day; approximately 0.025 to approximately 35 µg/kg/day; approximately 0.025 to approximately 30 µg/kg/day; approximately 0.025 to approximately 25 µg/kg/day; approximately 0.025 to approximately 20 µg/kg/day; and approximately 0.025 to approximately 15 µg/kg/day. In another embodiment, the dosage of sulfasalazine is from approximately 0.05 to approximately 15 µg/kg/day. In another embodiment, the dosage of sulfasalazine is from approximately 0.05 to approximately 10 µg/kg/day. In another embodiment, the dosage of sulfasalazine is from approximately 0.05 to approximately 5 µg/kg/day. In another embodiment, the dosage of sulfasalazine is from approximately 0.05 to approximately 20 µg/kg/day.

In one embodiment of the present invention, the NFκB inhibitor is sulindac and the dosage is from approximately 0.001 to approximately 100 µg/kg/day. Additional dosages of sulindac can include from approximately 0.001 to approximately 95 µg/kg/day; approximately 0.001 to approximately 90 µg/kg/day; approximately 0.001 to approximately 85 µg/kg/day; approximately 0.001 to approximately 80 µg/kg/day; approximately 0.001 to approximately 75 µg/kglday; approximately 0.025 to approximately 70 µg/kg/day; approximately 0.025 to approximately 65 µg/kg/day; approximately 0.025 to approximately 60 µg/kg/day; approximately 0.025 to approximately 55 µg/kg/day; approximately 0.025 to approximately 50 µg/kg/day; approximately 0.025 to approximately 45 µg/kg/day; approximately 0.025 to approximately 40 µg/kg/day; approximately 0.025 to approximately 35 µg/kg/day; approximately 0.005 to approximately 30 µg/kg/day; approximately 0.005 to approximately 25 µg/kg/day; approximately 0.005 to approximately 20 µg/kg/day; and approximately 0.005 to approximately 15 µg/kg/day. In another embodiment, the dosage of sulindac is from approximately 0.01 to approximately 15 µg/kg/day. In another embodiment, the dosage of sulindac is from approximately 0.01 to approximately 10 µg/kg/day. In another embodiment, the dosage of sulindac is from approximately 0.01 to approximately 5 µg/kg/day. In another embodiment, the dosage of sulindac is from approximately 0.01 to approximately 20 µg/kg/day.

The NFκB Inhibiting compounds provided herein may be administered in a physiologically acceptable carrier to a host, as previously described. In one method sustained release vehicles are utilized. The compositions may be administered in conjunction with other compositions for treatment.

The present invention also includes kits. In one embodiment, the kits of the present invention comprise NFκB inhibitors of the present invention. In another embodiment, a kit of the present invention can contain one or more of the following in a package or container (1) one or more NFκB inhibitors of the present invention; (2) one or more pharmaceutically acceptable adjuvants or excipients; (3) one or more vehicles for administration such as one or more syringes; (4) once or more additional bioactive agents for concurrent or sequential administration; (5) instructions for administration; and/or (6) a catheter and drug pump. Embodiments in which two or more of components (1) - (6) are found in the same container can also be used.

When a kit is supplied, the different components of the compositions included can be packaged in separate containers and admixed immediately before use. Such packaging of the components separately can permit long-term storage without losing the active components' functions. When more than one bioactive agent is included in a particular kit, the bioactive agents may be (1) packaged separately and admixed separately with appropriate (similar or different) vehicles immediately before use, (2) packaged together and admixed together immediately before use or (3) packaged separately and admixed together immediately before use. If the chosen compounds will remain stable after admixture, however, the admixture need not occur immediately before use but can occur at a time before use, including in one example, minutes, hours, days, months or years before use or in another embodiment at the time of manufacture.

The compositions included in particular kits of the present invention can be supplied in containers of any sort such that the life of the different components are preserved and are not adsorbed or altered by the materials of the container. For example, sealed glass ampules can contain lyophilized agents or variants or derivatives thereof or other bioactive agents, or buffers that have been packaged under a neutral, non-reacting gas, such as, without limitation, nitrogen. Ampules can consist of any suitable material, such as, without limitation. glass. organic polymers, such as, polycarbonate, polystyrene, etc., ceramic, metal or any other material typically employed to hold similar reagents. Other examples of suitable containers include, without limitation, simple bottles that may be fabricated from similar substances as ampules, and envelopes, that can comprise foil-lined interiors, such as aluminum or an alloy. Other containers include, without limitation, test tubes, vials, flasks, bottles, syringes, or the like. Containers can have one or more sterile access ports, such as a bottle having a stopper that can be pierced by a hypodermic injection needle. Other containers may have two compartments that are separated by a readily removable membrane that upon removal permits the components to be mixed. Removable membranes may be, without limitation, glass, plastic, rubber, etc.

As stated earlier, kits can also be supplied with instructional materials. Instructions may be printed on paper or other substrate, and/or may be supplied as an electronic-readable medium, such as a floppy disc, CD-ROM, DVD-ROM, Zip disc, videotape, audiotape, flash memory device, etc. Detailed instructions may not be physically associated with the kit; instead, a user may be directed to an internet web site specified by the manufacturer or distributor of the kit, or supplied as electronic mail.

The terms "a" and "an" and "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is herein deemed to contain the group as modified thus fulfilling the written description of all Markush groups used in the appended claims.

Certain embodiments of this invention are described herein, including the best mode known to the inventors for carrying out the invention. Of course, variations on these certain embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents and printed publications throughout this specification. Each of the above cited references and printed publications are herein individually incorporated by reference in their entirety.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

## Claims

1. An NFκB inhibitor selected from sulfasalazine, or sulindac, or combinations thereof, for use in the treatment of pain associated with sciatica or herniated disc, or radicular pain by local administration.

2. The pharmaceutical composition for use according to claim 1, wherein the use is at a dosage not exceeding 100 µg/kg/day.

3. The pharmaceutical composition for use according to claim 1, wherein the use is by local administration to:
(i) the perispinal region of the spine; or
(ii) the foraminal, epidural or intrathecal space of a spinal cord.

4. The pharmaceutical composition for use according to claim 1, wherein the use is with a catheter and drug pump, a syringe and/or polymer release.

5. A pharmaceutical composition comprising an NFκB inhibitor selected from sulfasalazine or sulindac, or combinations thereof, for use in the treatment of pain associated with sciatica or herniated disc, or radicular pain by local administration.

## Patentansprüche

1. NFκB-Inhibitor, der aus Sulfasalazin oder Sulindac oder Kombinationen davon ausgewählt ist, zur Verwendung bei der Behandlung von Schmerz, der mit Ischias oder Bandscheibenvorfall verbunden ist, oder von radikulärem Schmerz durch lokale Verabreichung.

2. Pharmazeutische Zusammensetzung zur Verwendung nach Anspruch 1, worin die Verwendung bei einer Dosierung von nicht mehr als 100 µg/kg/Tag erfolgt.

3. Pharmazeutische Zusammensetzung zur Verwendung nach Anspruch 1, worin die Verwendung durch lokale Verabreichung an Folgendem erfolgt:
(i) der perispinalen Region der Wirbelsäule; oder
(ii) dem foraminären, epiduralen oder intrathekalen Bereich eines Rückenmarks.

4. Pharmazeutische Zusammensetzung zur Verwendung nach Anspruch 1, worin die Verwendung mit einem Katheter und einer Arzneimittelpumpe, einer Spritze und/oder durch Polymer-Freisetzung erfolgt.

5. Pharmazeutische Zusammensetzung, die einen NFκB-Inhibitor umfasst, der aus Sulfasalazin oder Sulindac oder Kombinationen davon ausgewählt ist, zur Verwendung bei der Behandlung von Schmerz, der mit Ischias oder Bandscheibenvorfall verbunden ist, oder von radikulärem Schmerz durch lokale Verabreichung.

## Revendications

1. Inhibiteur de NFκB choisi parmi la sulfasalazine et le sulindac ou des combinaisons de ceux-ci, destiné à être utilisé dans le traitement de la douleur associée à une sciatique ou une hernie discale, ou d'une douleur radiculaire par administration locale.

2. Composition pharmaceutique destinée à être utilisée conformément à la revendication 1, dans laquelle l'utilisation se fait à un dosage ne dépassant pas 100 µg/kg/jour.

3. Composition pharmaceutique destinée à être utilisée conformément à la revendication 1, dans laquelle l'utilisation se fait par administration locale à :
(i) la région péri-vertébrale de la colonne vertébrale ; ou
(ii) l'espace foraminal, épidural ou intrathécal d'une moelle épinière.

4. Composition pharmaceutique destinée à être utilisée conformément à la revendication 1, dans laquelle l'utilisation se fait au moyen d'un cathéter et d'une pompe à médicament, d'une seringue et/ou d'une libération par un polymère.

5. Composition pharmaceutique comprenant un inhibiteur de NFκB choisi parmi la sulfasalazine et le sulindac ou des combinaisons de ceux-ci, destiné à être utilisé dans le traitement de la douleur associée à une sciatique ou une hernie discale, ou d'une douleur radiculaire par administration locale.
